Europäisches Patentamt

European Patent Office

Office européen des brevets

(11) Publication number: **0 436 858 A1**

(12) # EUROPEAN PATENT APPLICATION

(21) Application number: 90123877.4

(51) Int. Cl.5: **C12P 21/02, C12N 15/80**

(22) Date of filing: **12.12.90**

The title of the invention has been amended (Guidelines for Examination in the EPO, A-III, 7.3).

(30) Priority: **15.12.89 JP 326473/89**

(43) Date of publication of application:
**17.07.91 Bulletin 91/29**

(84) Designated Contracting States:
**BE CH DE DK ES FR GB IT LI NL SE**

(71) Applicant: **THE GREEN CROSS CORPORATION**
**3-3, Imabashi 1-chome Chuo-ku**
**Osaka-shi Osaka 541(JP)**

(72) Inventor: **Yukimitsu, Nakagawa, c/o The Green Cross**
**Corp. Centr. Research Lab., 2-1180-1,**
**Shodaiohtani**
**Hirakata-shi, Osaka 573(JP)**
Inventor: **Koji, Murakami, c/o The Green Cross**
**Corp. Centr. Research Lab., 2-1180-1,**
**Shodaiohtani**
**Hirakata-shi, Osaka 573(JP)**
Inventor: **Yutaka, Ishida, c/o The Green Cross**

**Corp. Centr. Research Lab., 2-1180-1,**
**Shodaiohtani**
**Hirakata-shi, Osaka 573(JP)**
Inventor: **Masanori, Morita, c/o The Green**
**Cross**
**Corp. Centr. Research Lab., 2-1180-1,**
**Shodaiohtani**
**Hirakata-shi, Osaka 573(JP)**
Inventor: **Muneo, Tsujikawa, c/o The Green**
**Cross**
**Corp. Centr. Research Lab., 2-1180-1,**
**Shodaiohtani**
**Hirakata-shi, Osaka 573(JP)**
Inventor: **Haruhide, Kawabe, c/o The Green**
**Cross**
**Corp. Centr. Research Lab., 2-1180-1,**
**Shodaiohtani**
**Hirakata-shi, Osaka 573(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Promoter of glyceraldehyde-3-phosphate dehydrogenase gene and its use.**

(57) A method of producing a foreign protein with mold such as yellow-green koji mold or black koji mold, characterized by the use of a GAP-DH promoter derived from yellow-green koji mold or black koji mold is described. The present invention makes a new production technique based on gene recombination technology possible. Accordingly, the present invention will contribute significantly to production of useful substances using a mold, especially a yellow-green koji mold host-vector system.

EP 0 436 858 A1

## PRODUCTION METHOD FOR FOREIGN PROTEIN

BACKGROUND OF THE INVENTION

The present invention provides a method of efficiently produce a foreign protein by gene recombination technology using mold (e.g. yellow-green koji mold, black koji mold, etc.) as the host. The present invention is characterized by the use of a GAP-DH promoter derived from yellow-green koji mold or black koji mold in the expression system.

Yeast promoter GAP-DH, already in public knowledge, is used in production plasmids for a wide variety of foreign proteins such as hepatitis B virus surface antigen (HBsAg) [Bitler, G. A. and Egou, K. M.: Gene, *32*, 263-274 (1984)]. For example, pGG5, a plasmid for HBsAg production, was constructed with a GAP-DH promoter, an HBsAg gene, a GAP-DH terminator, an *Eschelichia coli* replication origin, a marker gene and a yeast replication origin (EP Publication No. 218209-A).

Yellow-green koji mold and black koji mold have recently become used as hosts for gene recombination technology, and little has been studied on their expression system. Therefore, it remains unknown which types of promoter this fungal group has and which types of expression system are efficient in the expression of a foreign protein in this host system.

The object of the present invention is to obtain a promoter specific to the host system from yellow-green koji mold or black koji mold and providing a method of producing a foreign protein using this expression system.

SUMMARY OF THE INVENTION

The present invention comprises a method of producing a foreign protein with mold (e.g. yellow-green koji mold, black koji mold, etc. ) characterized by the use of a GAP-DH promoter derived from yellow-green koji mold or black koji mold.

The present invention is novel in that mold (e.g. yellow-green koji mold, black koji mold, etc. ) is used as the host and a GAP-DH promoter derived from yellow-green koji mold is used as the promoter.

BRIEF DESCRIPTION OF THE DRAWINGS

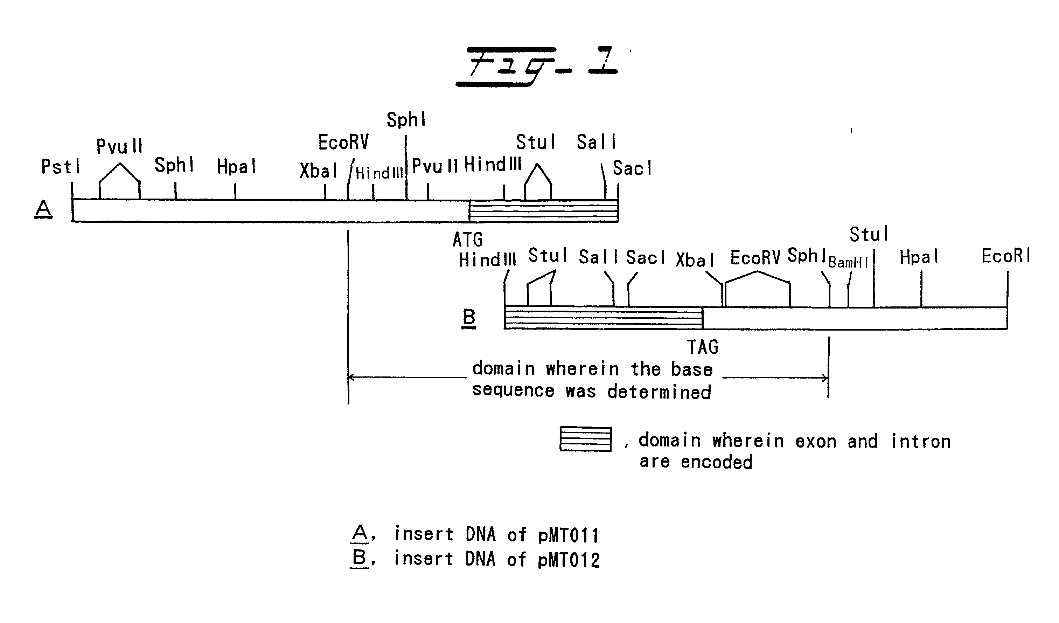

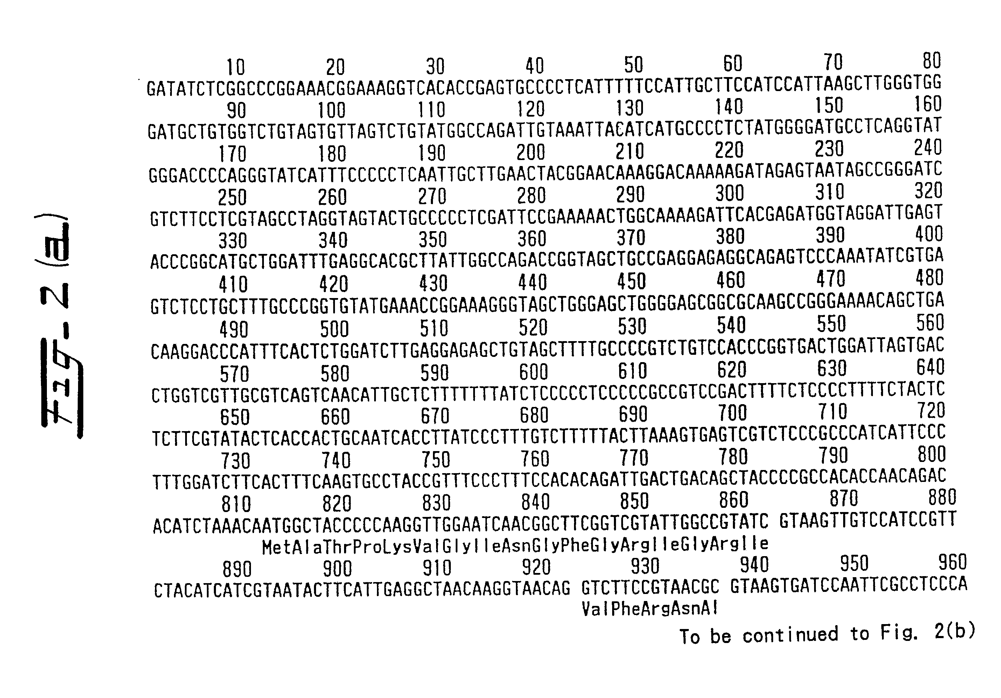

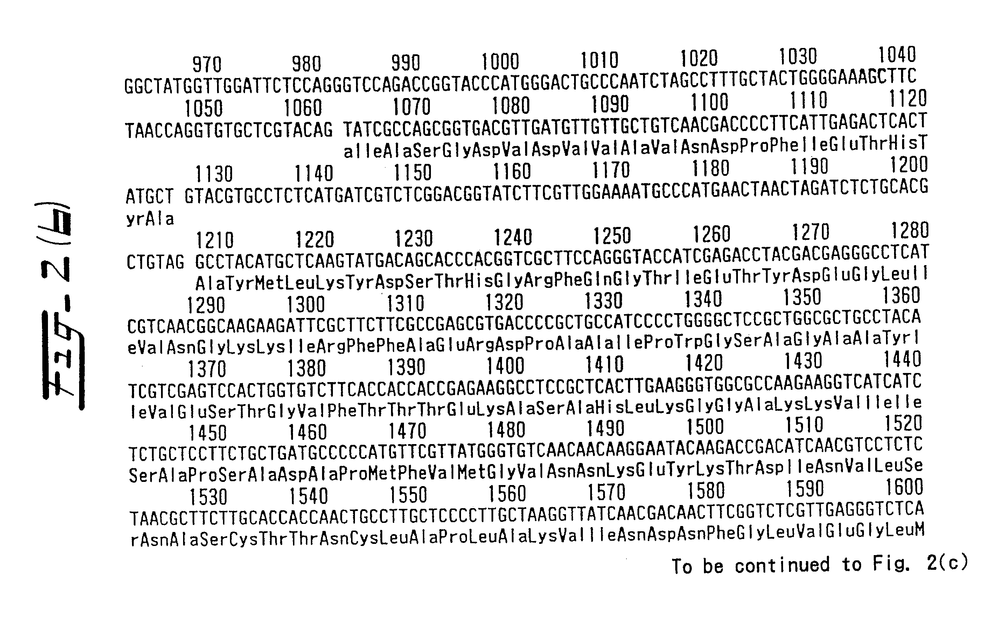

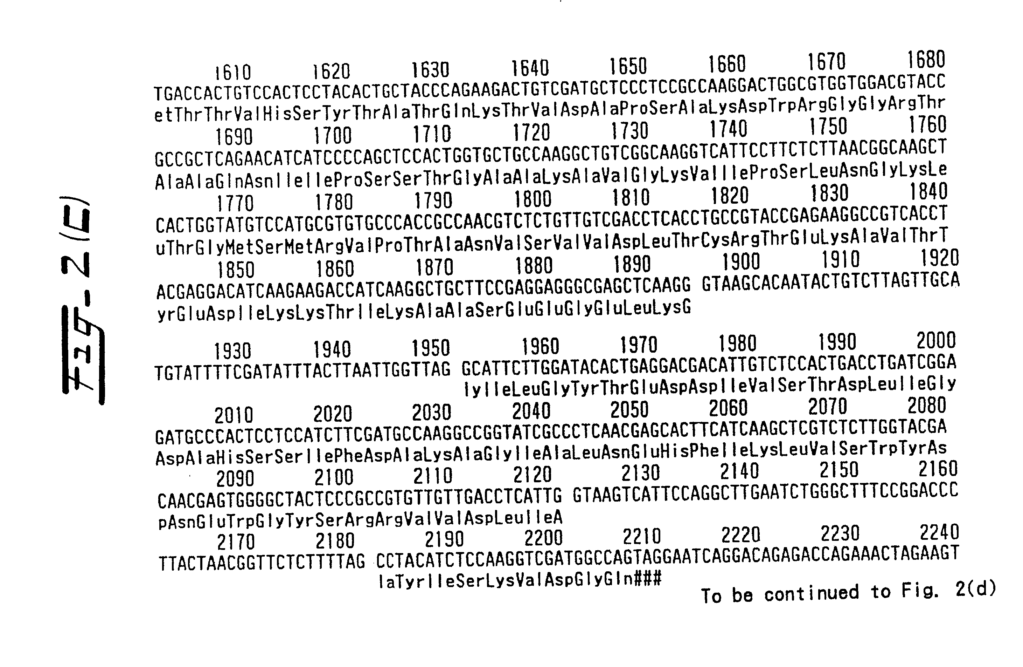

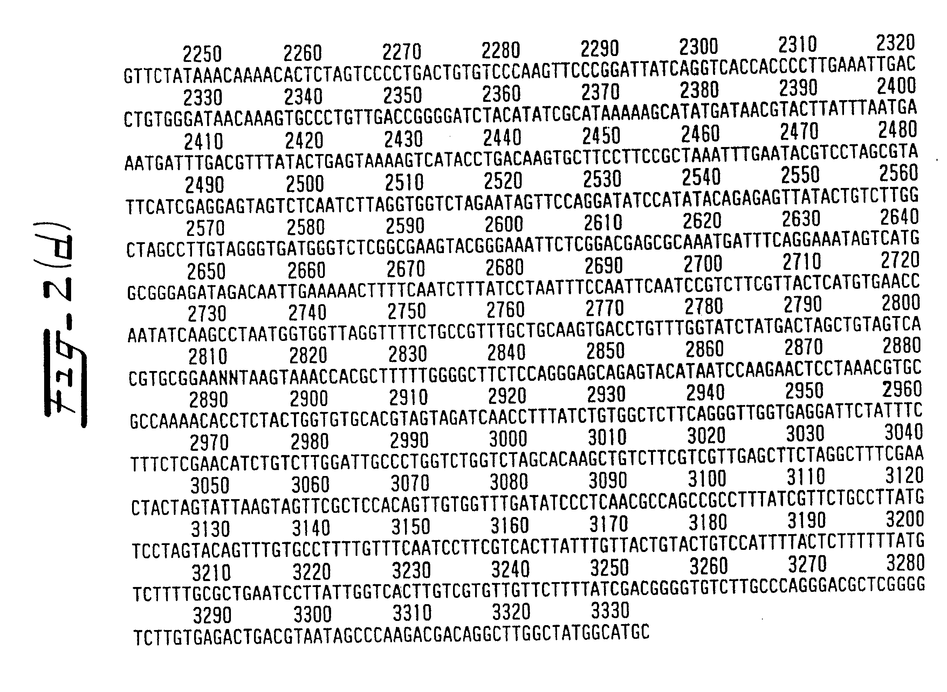

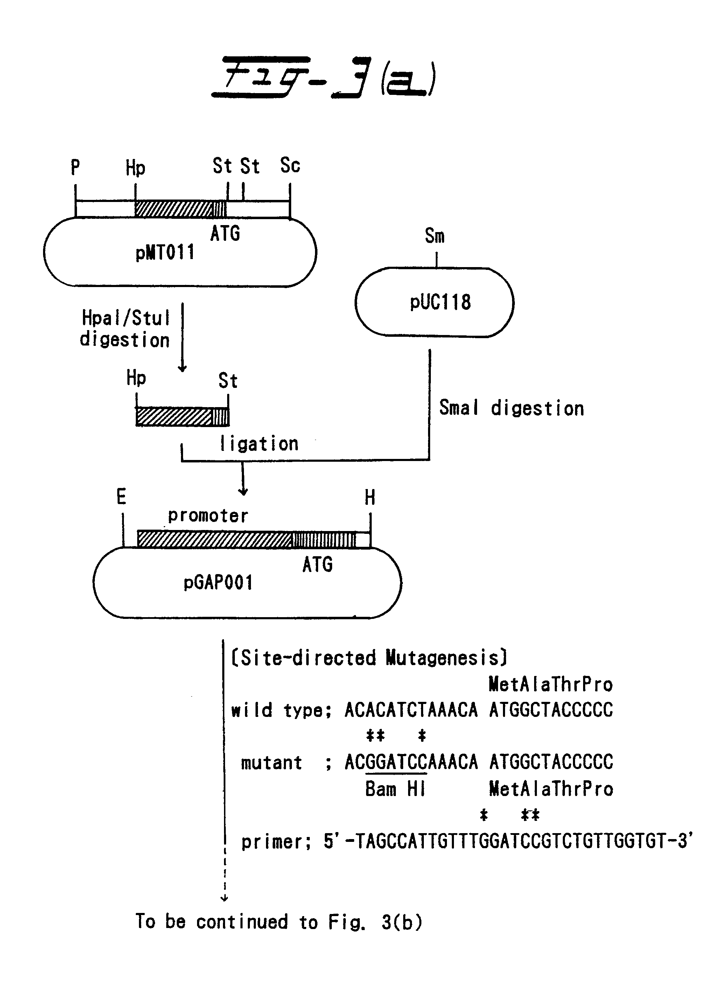

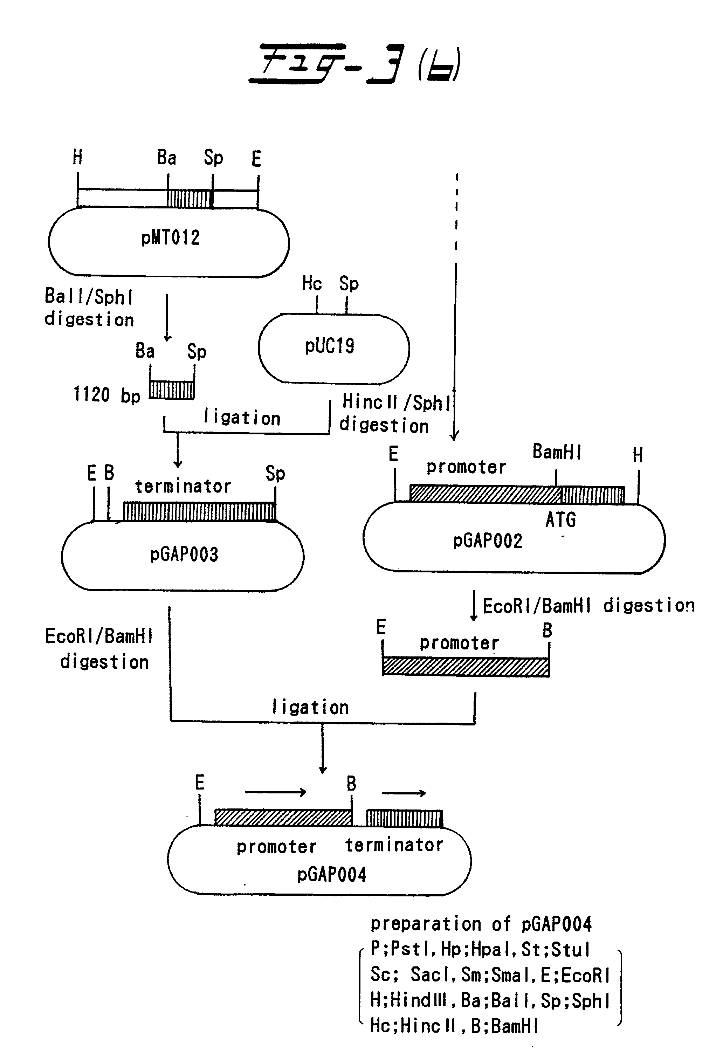

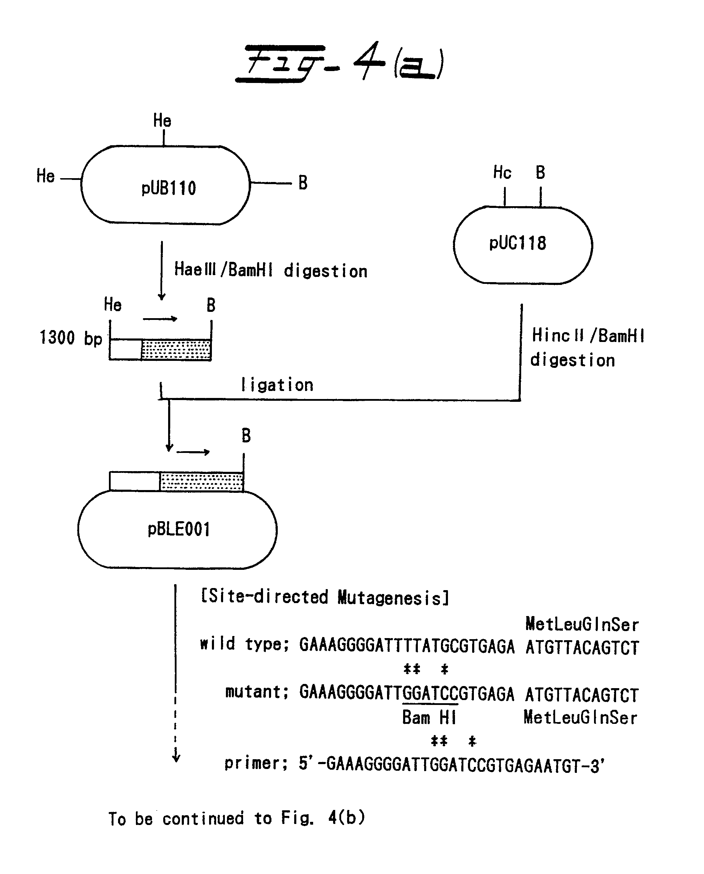

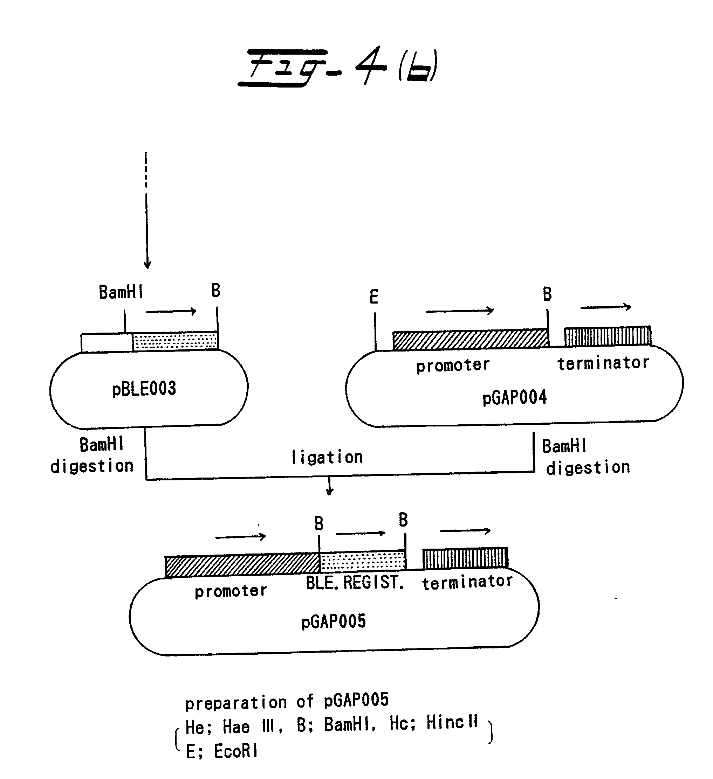

Figure 1 shows the restriction enzyme maps of the insert DNA of pMT011 and pMT012. Figure 2 (a) and (b) show the base sequence of the *A. oryzae* GAP-DH gene and the amino acid sequence encoded thereby. Figure 3 shows the construction schematic for pGAP004. Figure 4 shows the construction schematic for pGAP005.

DETAILED DESCRIPTION OF THE INVENTION

In the present invention, a known yeast-derived GAP-DH (glyceraldehyde-3-phosphate dehydrogenase) promoter was prepared as a probe, and a gene library of chromosome DNA extracted from yellow-green koji mold or black koji mold was prepared and then extracted using the probe described above. Then, using a plasmid containing a GAP-DH promoter domain, an expression vector incorporating a foreign protein structural gene and a marker gene was constructed. This vector was used to transform mold (e.g. yellow-green koji mold, black koji mold, etc. ) to yield the desired foreign protein.

In the present invention, the yeast GAP-DH promoter used as the probe was prepared in accordance with Reference Example 1 described below, using a fragment of plasmid pYN019, which has a yeast GAP-DH promoter domain (containing UAS, CAAT box and TATA box). Extraction of the chromosome DNA from yellow-green koji mold or black koji mold was achieved by deproteinization with phenol followed by ethanol precipitation. The yellow-green koji mold or black koji mold used as a starting material was ATCC 42149 strain (*Aspergillus oryzae* ). A gene library was prepared by ligating a chromosome DNA fragment which possibly had a part of the GAP-DH translation domain of yellow-green koji mold or black koji mold and a non-translation domain adjacent to the translation domain to an appropriate plasmid.

The gene library thus obtained was screened by the colony hybridization method using the yeast GAP-DH translation domain probe described above, and a positive clone was obtained.

A partial base sequence of this clone was determined, and plasmid pMT011, containing a 5' side non-translation domain and N-terminal side translation domain of *A. oryzae* GAP-DH, was obtained.

Separately, another plasmid pMT012, containing a C-terminal side translation domain and 3' side non-translation domain of *A. oryzae* GAP-DH, was obtained from a positive clone obtained in the same manner as above, confirmed by comparing with the GAP-DH sequence of *A. nidulans*. The base sequence of a fragment of this plasmid, containing a GAP-DH promoter domain of yellow-green koji mold or black koji mold was determined and compared with the gene of *A.nidulans* [Punt, P. J., Dingemanse, M, A., Jacobs-Meijsing, B. J. M., Pouwels, P. H. and van den Hondel: C. A. M. J. I.,

Gene, *69*, 49-57 (1988)]; it was confirmed that they are very homologous to each other. Then, a restriction enzyme site for foreign gene insertion was made downstream the plasmid containing the GAP-DH promoter domain cleaved out from pMT011, and the GAP-DH terminator domain obtained from pMT012 was inserted thereinto.

The plasmid thus obtained can be used as a unit for gene expression. It provides a vector expressible in yellow-green koji mold or black koji mold by inserting an appropriate foreign gene into said restriction enzyme site for foreign gene insertion before use.

In the working examples of the present invention, the bleomycin resistance gene derived from *Staphylococcus aureus* was used as a foreign gene. Of course, this gene may be a gene of a bioactive substance such as human urokinase, human TPA, human albumin or a kind of interferon.

Next, the present invention provides a method of producing a foreign protein by transforming mold (e.g. yellow-green koji mold, black koji mold, etc. ) with the above-mentioned vector. Examples of the yellow-green koji mold used include *A. oryzae* and *A. sojae*; examples of the black koji mold used include *A. niger* and *A.awamori*.

The present invention provides a method of efficiently producing a foreign protein in mold (e.g. yellow-green koji mold, black koji mold, etc.) and makes a new production technique based on gene recombination technology possible. Accordingly, the present invention will contribute significantly to production of useful substances using a mold, especially a yellow-green koji mold host-vector system. The present invention is hereinafter described in more detail by means of the following working examples, but these are not to be construed as limitative.

Example 1

Preparation of probes for cloning of yellow-green koji mold GAP-DH promoter and terminator genes

After $20\mu$g of pYN019, having a yeast GAP-DH promoter domain (containing UAS, CAAT box and TATA box), was digested with EcoRI and BamHI, 1% low melting point gel electrophoresis was carried out, and a 0.65 kbp fragment which encodes the yeast GAP-DH promoter domain was cleaved out and subjected to phenol extraction and ethanol precipitaion to recover a DNA (yield $2\mu$g).

After $20\mu$g of pGAP301, having a 5' side non-translation domain, translation domain and 3' side non-translation domain of yeast GAP-DH was digested with XbaI and SalI, 1% low melting point gel electrophoresis was carried out, and a 0.96 kbp fragment which encodes the yeast GAP-DH translation domain was recovered (yield 800 ng). This DNA was labeled with a NICK translation kit (produced by Takara Shuzo) in accordance with the protocol for the kit. The [$\alpha$ -$^{32}$P]dCTP used was 3000 Ci/mmol PB10205, produced by Amersham Corporation. The labeled probes were purified using a Nick column (produced by Pharmacia). Probe 1, obtained by labeling the 0.65 kb fragment, and Probe 2, obtained by labeling the 0.9 kb fragment, had a specific activity of $1.68 \times 10^8$ cpm/ $\mu$g or $2.67 \times 10^8$ cpm/ $\mu$g, respectively.

Example 2

Extraction of chromosome DNA of *A. oryzae*

One platinum loopful of *Aspergillus oryzae* ATCC 42149 strain was inoculated to a 300-ml conical flask containing 50 ml of YPD medium (1% yeast extract, 2% bacto peptone, 2% dextrose) and subjected to shaking culture at 30°C for one day. The resulting preculture broth was transferred to two 3-liter conical flasks each containing 500 ml of YPS medium (1% yeast extract, 2% bacto peptone, 2% soluble starch) in an amount of 25 ml for each flask and subjected to shaking culture at 30°C for one day. Cells were harvested from this culture broth (1 $\ell$ ) to yield 32.5 g (wet weight of fungal cells. After being milled in a mortar, the cells were deproteinized with phenol and precipitated with ethanol to extract the chromosome DNA. Eventually, $860\mu$g of DNA was obtained from 5 g of cells.

Examples 3

(a) Preparation of gene library of 4 kbp EcoRI-HindIII fragment

$86\mu$g of yellow-green koji mold chromosome DNA was digested with HindIII overnight. After ethanol precipitation and drying, the chromosome DNA was further digested with EcoRI for 6 hours, followed by 0.8% low melting point agarose gel electrophoresis. An about 3.4 kpb to 4.8 kpb band was cut out and subjected to phenol extraction and ethanol precipitation to recover the DNA. Also, $5\mu$g of pUC18 was digested with EcoRI and HindIII overnight and subjected to ethanol precipitation and drying, followed by reaction with 4 units of calf small intestine derived alkaline phosphatase (CIP) at 37°C for 30 minutes and then at 56°C for 30 minutes. Then, a 2.7 kbp fragment was cleaved off via 0.8% low melting point agarose gel electrophoresis and subjected to phenol extraction and ethanol precipitation to recover the DNA.

To $7\mu$l of a solution containing $2\mu$g of the 3.4-4.8 kbp fragment of chromosome DNA and $1\mu$g of

the CIP-treated pUC18 EcoRI-HindIII fragment, 56μl of ligation kit (produced by Takara Shuzo) Solution A and 7μl of Solution B were added, and reaction was carried out at 16°C overnight to transform competent cells Escherichia coli HB101 (produced by Takara Shuzo) therewith to yield 90,000 transformants. From 24 of these transformants, plasmid DNA was extracted by a simple extraction method and examined for insert DNA. All examined transformants were found to contain an insert DNA.

(b) Screening by colony hybridization

Using $^{32}$P-labeled Probe 2, 15,000 clones were screened by colony hybridization and 50 signals were found. From 17 of these signals, plasmid DNA was prepared for 19 clones and digested with BamHI + HindIII. A 2.5 kbp (nearly the expected value) band appeared in 13 clones (13 signals). Out of them, 4 clones were digested with EcoRI + HindIII, HindIII + Xba1 and EcoRI + Xba1, and the band sizes were found to be almost the same as the expected values, suggesting that these clones are probably the desired clones.

The base sequence of this clone was determined in the 247 bp portion from the HindIII site (a portion homologous with the yeast GAP-DH translation domain probe). This sequence was examined for homology with the base sequence of A.nidulans GAP-DH (Punt et al., mentioned above). It was found that an 84% homology exists between the base sequence of base Nos. 165 to 230 as counted from the HindIII site and the 584-660 base sequence of A. nidulans GAP-DH gene described by Punt et al. (Gene, mentioned above) (a domain corresponding to exon VI). From these findings, it was speculated that the 4 kbp HindIII-EcoRI fragment contains a C-terminal side translation domain and 3' side non-translation domain of GAP-DH. This clone was named pMT012 (See Figure 1).

Example 4

(a) Preparation of gene library of 4.1 kbp SacI-PstI fragment

86μg of yellow-green koji mold chromosome DNA was digested with SacI overnight and subjected to ethanol precipitation and drying, after which it was further digested with PstI for 6 hors and subjected to 0.8% low melting point agarose gel electrophoresis. An about 3.4-4.8 kbp band was cut out and subjected to phenol extraction and ethanol precipitation to recover the DNA. Sepatately, 5μg of pUC 18 was digested with SacI and PstI overnight and subjected to ethanol precipitaion and drying, followed by reaction with 4 units of CIP at 37°C for 30 minutes and then at 56°C for 30 minutes. Then 4 units of CIP was further added and reaction was carried out at 37°C for 30 minutes and then at 56°C for 30 minutes. Then, a 2.7 kbp fragment was cleaved out by 0.8% low melting point agarose gel electrophoresis and subjected to phenol extraction and ethanol precipitation to recover the DNA.

To 7μl of a solution containing 2μg of the 3.4-4.8 kbp fragment of chromosome DNA and 1μg of the CIP-treated pUC18 EcoRI-HindIII fragment, 56μl of ligation kit Solution A and 7μl of Solution B were added, and reaction was carried out at 16°C overnight to transform competent cells HB101 therewith to yield 60,000 transformants. From 24 of these transformants, plasmid DNA was prepared by a simple extraction method and examined for insert DNA. 23 of the examined transformants were found to contain an insert DNA.

(b) Screening by colony hybridization

Using $^{32}$P-labeled Probe 1, 3,500 clones were screened by colony hybridization, and 12 signals were found. From these 12 signals, plasmid DNA was prepared for 25 clones by a simple extraction method and digested with HindIII; a 3.5 kbp (nearly the expected value) band appeared in 2 clones (2 signals). They were further digested with SphI, SacI, PstI, EcoRI + HpaI, and EcoRI + XbaI, and the band sizes were found to be almost the same as the expected values, suggesting that these clones are probably the desired clones.

The base sequence of this clone was determined in the 200bp portion from the SacI site (a portion homologous with the yeast GAP-DH translation domain probe). This sequence was examined for homology with the base sequence of the A. nidulans GAP-DH (Punt et al., mentioned above). It was found that a 79% homology exists between the base sequence of a complementary chain of this sequence and the 1069-1266 base sequence of A. nidulans GAP-DH gene (a domain corresponding to the C-terminal side of exon VI) described by Punt et al. (Gene, mentioned above). From these findings, it was speculated that the 4 kbp PstI-SacI fragment contains an N-terminal side translation domain and 5' side non-translation domain of GAP-DH. This clone was named pMT011 (See Figure 1).

A partial base sequence of the insert DNA of pMT011 and pMT012 was determined. Results are shown in Figure 2.

Example 5

Preparation of expression vector incorporating GAP-DH gene promoter and terminator

A yellow-green koji mold expression vector incorporating a GAP-DH gene promoter and terminator was prepared as follows: From plasmid pMT011, wherein an N-terminal side domain of the GAP-DH gene has been cloned, the promoter domain was cleaved out using restriction enzymes HpaI and StuI and mixed with an SmaI (produced by Takara Shuzo) digestion product of pUC118 (produced by Takara Shuzo). After ligation, the mixture was introduced into *Escherichia coli* JM109 strain competent cell (produced by Takara Shuzo) to yield plasmid pGAP001. Then, to make a restriction enzyme site for foreign gene insertion (BamHI recognition site in the present invention) downstream this promoter domain, site directed mutagenesis was conducted as shown in Figure 3. The synthetic oligonucleotide used for the site directed mutagenesis was synthesilzed using the DNA Synthesizer model 381A, produced by Applied Biosystems. Site specific mutation can be conducted using the Oligonucleotide-directed In Vitro Mutagenesis System version 2, produced by Amersham Corporation. This operation permits us to prepare plasmid pGAP002, having a DNA wherein a BamHI recognition site is provided 5 base upstream the ATG initiation codon of the GAP-DH gene.

Separately, the terminator domain was isolated by digesting pMT012 with BalI and SphI (produced by Takara Shuzo). A 1120 bp DNA fragment containing this terminator domain was inserted into pUC19 at the Hinc II/SphI site to yield plasmid pGAP003. This pGAP003 was digested with EcoRI/BamHI to yield a vector DNA, which was mixed with a GAP-DH promoter fragment obtained by digesting pGAP002 with EcoRI/BamHI, followed by ligation and *Escherichia coli* JM109 strain transformation to yield pGAP004. This plasmid pGAP004 is a plasmid wherein the GAP-DH gene promoter and terminator are present serially, and is expressible in yellow-green koji mold by inserting an appropriate foreign gene at the BamHI recognition site present therebetween.

Example 6

Preparation of bleomycin resistance gene expression vector

The bleomycin resistance gene is encoded in *Escherichia coli* transposon Tn5 and *Staphylococcus aureus* derived plasmid pUB110 [Semon et al.: Plasmid, *17*, 46-53 (1987)]. It is already known that the bleomycin resistance gene derived from transposon Tn5 functions in *Escherichia coli* and yeasts (Gatignol et al (1987) Mol. Gen. Genet. *207*, 342-348) and that the bleomycin resistance gene derived from pUB110 functions in *Escherichia coli* (Semon et al., mentioned above); however, none of these resistance genes has been reported to function in yellow-green koji mold. In the present example, a vector was prepared to express the pUB110-derived bleomycin resistance gene using the GAP-DH gene promoter and terminator, and a transformant obtained by transformation with this vector proved to have gained bleomycin resistance.

The bleomycin resistance gene expression vector was prepared in accordance with Figure 4. First, the bleomycin resistance gene encoded in pUB110 was obtained as an about 1300 bp DNA fragment by digestion with restriction enzymes HaeIII/BamHI. This fragment was inserted into *Escherichia coli* plasmid pUC118 (produced by Takara Shuzo) at the HincII/BamHI site to yield plasmid pBLE001. Although pBLE001 contains the bleomycin resistance gene, it is impossible to directly insert it into the expression vector pGAP004 because of the absence of a restriction enzyme recognition site at an appropriate position upstream its ATC initiation codon. Thus, site-directed mutagenesis was conducted to provide a restriction enzyme recognition site (BamHI in the present example) at an appropriate position. The design and primer sequence for the site-directed mutagenesis are given in Figure 4. Site-directed mutagenesis can be carried out using the above-mentioned Oligonucleotide-directed In Vitro Mutagenesis System version 2, produced by Amersham Corporation. In the pBLE002 thus prepared, a BamHI site is present at the position of 6 bp upstream the ATG initiation codon of the bleomycin resistance gene. This pBLE002 was digested with BamHI, and the bleomycin resistance gene was cleaved out. This DNA fragment was inserted into pGAP004 at the BamHI site to yield pGAP005. pGAP005 is a vector capable of expressing the pUB110-derived bleomycin resistance gene under control of the yellow-green koji mold GAP-DH gene promoter and terminator.

Example 7

Transformation of yellow-green koji mold *A. oryzae* with pGAP005

Examples of selection markers which have been used to transform *A. oryzae* include argB, amdS [Christensen et al.: BIO/TECHNOLOGY, *6*, 1419-1422 (1988)], pyrG [Mattern et al.: Mol. Gen. Genet., *210*, 460-461 (1987)], met [Iimura et al.: Agric. Biol. Chem., *51*, 323-328 (1987)]and niaD [Unkels et al.: Mol. Gen. Genet., *218*, 99-104 (1989)]. However, in considering transformation of *A. oryzae* as an industrial microbe, a dominant marker is essential, but at present the amdS system using acetamide utilizability as a marker alone is available. It is therefore important to develop a transformation system for drug resistance etc. In the present example, it has been demonstrated that the *A. oryzae* strain incorporating pGAP005, the bleomycin resistance expression vector incorporating the GAP-DH gene promoter and terminator, prepared in the previous term, is capable of gaining bleomycin resistance.

The *A. oryzae* transformation was achieved by a modification of the method of Iimura et al. (Iimura et al., mentioned above). The hosts used were bleomycin sensitive strains *A. oryzae* IFO 4177 and IFO 4181. Each strain was cultured on a slant [MD medium: 0.2% malt extract (Difco Laboratories), 0.2% dextrose (produced by Nacalai Tesque), 0.01% Bacto Peptone (produced by Difco Laboratories), 2.0% agar (produced by Nacalai Tesque)]at 30°C for 4 to 5 days until spores were formed. The formed spores were suspended in sterile water to yield a spore suspension. This spore suspension was transferred into a 200-ml conical flask containing 30 ml of DP medium [2.0% dextrin (produced by Difco Laboratories), 1.0% Polypeptone (produced by Daigo Eiyo), 0.5% $kH_2PO_4$ (produced by Nacalai Tesque), 0.1% $NaNO_3$ (produced by Nacalai Tesque), 0.05% $MgSO_4 \cdot 7H_2O$ (produced by Nacalai Tesque)] in an amount of $10^7$ to $10^8$ spores, and subjected to shaking culture at 30°C overnight. Using a 3GI glass filter (produced by Iwaki Glass), cells were harvested from the culture broth, and washed twice with sterile water and once with a phosphate buffer (10 mM phosphate buffer, pH 6.0, 5% NaCl). Then, the cells were suspended in 10 ml of an enzyme solution [10 mM phosphate buffer, pH 6.0, 5% NaCl, 20 mg/ml Novozym 234 (produced by NOVO Co.), 2 mg/ml Chitinase (produced by Sigma Co. )]- and incubated at 30°C for 2 hours. After protoplast formation was confirmed microscopically, the protoplasts were collected into a centrifugal tube using a 3G2 glass filter (produced by Iwaki Glass). After pelletization by centrifugation at 1000×g for 5 minutes, the protoplasts were suspended and washed once with phosphate buffer (mentioned above) and then once with STC buffer [5% NaCl, 10 mM $CaCl_2$, 10 mM Tris-HCl (pH 7.5)], after which they were suspended in STC buffer to reach a final protoplast concentration of $1 \times 10^8$ protoplasts/ml. A 0.2 ml portion of this protoplast solution ($2 \times 10^7$ protoplasts) was transferred into a 15-ml centrifugal tube and mixed with a DNA solution (pGAP005, 5 to 50μg), and the mixture was kept standing at 0°C for 30 minutes. After adding 1 ml of a polyethylene glycol (PEG) solution [25% PEG4000, 10 mM $CaCl_2$, 10 mM Tris-HCl (pH 7.5)] and gentle stirring, the mixture was kept standing at room temperature for 15 minutes. To this mixture was added 5 ml of STC buffer (mentioned above), followed by centrifugation at 1000×g and supernatant removal. This procedure was repeated in one more cycle, and the protoplasts were washed and finally suspended in 1 ml of STC buffer. After mixing 200μl of this protoplast suspension with 5 ml of top agar (MD medium + 5% NaCl, 0.5% agar, incubated at 45°C), the mixture was layered on an MD plate (MD medium + 5% NaCl, 2.0% agar). After agar solidification, the plate was incubated at 30°C. After cultivation for 6 hours, 5 ml of a top agar (MD medium + 5% NaCl, 0.8% agar, incubated at 45°C) supplemented with bleomycin hydrochloride (produced by Nippon Kayaku Co. ) to a final concentration of 300μg/ml, was layered, follwed by cultivation at 30°C for 4 to 7 days. The colony which appeared was subcultured on an MD plate containing 300μg/ml bleomycin hydrochloride 3 to 4 times to yield a transformant.

Example 8

Analysis of the strain which became resistant to bleomycin

To determine whether the bleomycin-resistant strain obtained in the previous term was a pGAP005 transformant, the following analysis was made. First, the chromosome DNA of the obtained bleomycin-resistant strain was extracted and purified by the method described in Example 2. Next, after digestion with BamHI, these chromosomes were separated from each other by 1% agarose electrophoresis and subjected to Southern blotting onto a nitrocellulose filter. Southern blotting was conducted in accordance with the method of Maniatis et al. [Molecular Cloning, Cold Spring Harbor Laboratory, Cold Spring Harbor, NY, (1982)]. The probe used was a 1200 bp DNA fragment obtained by digesting pGAP005 with BamHI. This DNA fragment encodes the bleomycin resistance gene. This fragment was extracted and purified from the agarose gel and labeled with [$\alpha$-$^{32}$P] dCTP using the Random Primer DNA Labeling Kit (produced by Takara Shuzo) and used as a probe. Hybridization was conducted at a temperature of 65°C and a salt concentration of 6×SSC in accordance with the method of Maniatis et al. (mentioned above), followed by washing at 65°C and 0.1×SSC. As a result, in 3 of the 10 bleomycin-resistant strains, a band which hybridized with the probe in a size of about 1200 bp was detected. It was thus proven that these strains are transformants transformed with pGAP005.

These findings demonstrate that:
(1) the *A. oryzae* GAP-DH promoter and terminator cloned in the present example have activities and are capable of expressing a foreign gene, and

(2) the bleomycin resistance gene encoded by plasmid pUB110, derived from *Staphylococcus aureus,* functions in *A. oryzae* and can be used as a dominant marker in transformation.

## Claims

1. A method of producing a foreign protein with mold, characterized by the use of a GAP-DH promoter derived from yellow-green koji mold or black koji mold.

2. A method of producing a foreign protein as claimed in Claim 1, wherein the mold is a yellow-green koji mold or a black koji mold.

3. A method of producing a foreign protein as claimed in Claim 1, comprising constructing an expression vector incorporating a foreign protein structural gene and a marker gene with the use of a plasmid containing a GAP-DH promoter domain, transforming mold using the vector, culturing the obtained transformant, and harvesting the foreign protein from the culture broth.

4. A method of producing a foreign protein as claimed in Claim 1, wherein the GAP-DH promoter is derived from *A. orizae .*

5. A method of producing a foreign protein as claimed in Claim 1, wherein the foreign protein structural gene is one species selected from the group consisting of bleomycin resistance genes derived from *Staphylococcus aureus,* human urokinase, human TPA, human albumin and various interferons.

6. A GAP-DH promoter derived from *A. orizae.*

7. A vector capable of expressing a foreign protein gene with mold under control of a GAP-DH gene promoter and terminator.

8. A vector claimed in Claim 6, wherein the foreign protein gene is a bleomycin resistance gene.

*Fig-1*

A, insert DNA of pMT011
B, insert DNA of pMT012

domain wherein the base sequence was determined

, domain wherein exon and intron are encoded

EP 0 436 858 A1

## Fig. 2 (a)

```
         10        20        30        40        50        60        70        80
GATATCTCGGCCCGGAAACGGAAAGGTCACACCGAGTGCCCCTCATTTTTCCATTGCTTCCATCCATTAAGCTTGGGTGG
         90       100       110       120       130       140       150       160
GATGCTGTGGTCTGTAGTGTTAGTCTGTATGGCCAGATTGTAAATTACATCATGCCCCTCTATGGGGATGCCTCAGGTAT
        170       180       190       200       210       220       230       240
GGGACCCCAGGGTATCATTTCCCCCTCAATTGCTTGAACTACGGAACAAAGGACAAAAAGATAGAGTAATAGCCGGGATC
        250       260       270       280       290       300       310       320
GTCTTCCTCGTAGCCTAGGTAGTACTGCCCCCTCGATTCCGAAAAACTGGCAAAAGATTCACGAGATGGTAGGATTGAGT
        330       340       350       360       370       380       390       400
ACCCGGCATGCTGGATTTGAGGCACGCTTATTGGCCAGACCGGTAGCTGCCGAGGAGAGGCAGAGTCCCAAATATCGTGA
        410       420       430       440       450       460       470       480
GTCTCCTGCTTTGCCCGGTGTATGAAACCGGAAAGGGTAGCTGGGAGCTGGGGAGCGGCGCAAGCCGGGAAAACAGCTGA
        490       500       510       520       530       540       550       560
CAAGGACCCATTTCACTCTGGATCTTGAGGAGAGCTGTAGCTTTTGCCCCGTCTGTCCACCCGGTGACTGGATTAGTGAC
        570       580       590       600       610       620       630       640
CTGGTCGTTGCGTCAGTCAACATTGCTCTTTTTTTATCTCCCCCTCCCCCGCCGTCCGACTTTTCTCCCCTTTTCTACTC
        650       660       670       680       690       700       710       720
TCTTCGTATACTCACCACTGCAATCACCTTATCCCTTTGTCTTTTTACTTAAAGTGAGTCGTCTCCCGCCCATCATTCCC
        730       740       750       760       770       780       790       800
TTTGGATCTTCACTTTCAAGTGCCTACCGTTTCCCTTTCCACACAGATTGACTGACAGCTACCCCGCCACACCAACAGAC
        810       820       830       840       850       860       870       880
ACATCTAAACAATGGCTACCCCCAAGGTTGGAATCAACGGCTTCGGTCGTATTGGCCGTATC GTAAGTTGTCCATCCGTT
             MetAlaThrProLysValGlyIleAsnGlyPheGlyArgIleGlyArgIle
        890       900       910       920       930       940       950       960
CTACATCATCGTAATACTTCATTGAGGCTAACAAGGTAACAG GTCTTCCGTAACGC GTAAGTGATCCAATTCGCCTCCCA
                                              ValPheArgAsnAl
```

To be continued to Fig. 2(b)

9

# Fig. 2 (b)

```
                                                                                            
         970       980       990      1000      1010      1020      1030      1040
GGCTATGGTTGGATTCTCCAGGGTCCAGAGGTACCGGTACCGCCAATCTAGCCTTTGCTACTGGGGAAAGCTTC

        1050      1060      1070      1080      1090      1100      1110      1120
TAACCAGGTGTGCTCGTACAG TATCGCCAGCGGGTGACGTTGATGTTGTTGCTGTCAACGACCCCTTCATTGAGACTCACT
                      alleAlaSerGlyAspValAlaAlaValAsnAspProPheIleGluThrHisT

        1130      1140      1150      1160      1170      1180      1190      1200
ATGCT GTACGTGCCTCTCATGATCGTCTCGGACGGTATCTTCGTTGGAAAATGCCCATGAACTAACTAGATCTCTGCACG
yrAla

        1210      1220      1230      1240      1250      1260      1270      1280
CTGTAG GCCTACATGCTCAAGTATGACAGCACCACGGTCGCTTCCAGGGTACCATCGAGACCTACGACGAGGGCCTCAT
       AlaTyrMetLeuLysTyrAspSerThrHisGlyArgPheGlnGlyThrIleGluThrTyrAspGluGlyLeuIl

        1290      1300      1310      1320      1330      1340      1350      1360
CGTCAACGGCAAGAAGAATTCGCTTCTTCGCCGAGCGTGACCCCGCCATCCCCTGGGGCCTCCGGCTGCCTACA
eValAsnGlyLysLysIleArgPhePheAlaGluArgAspProAlaIleIleProTrpGlySerAlaGlyAlaTyr

        1370      1380      1390      1400      1410      1420      1430      1440
TCGTCGAGTCCACTGGTGTCTTCACCACCACCGAGAAGGCCTCACTTGAAGGTGGCGCGCCAAGAAGGTCATCATC
leValGluSerThrGlyValPheThrThrThrGluLysAlaSerAlaHisLeuLysGlyGlyAlaLysLysValIleIle

        1450      1460      1470      1480      1490      1500      1510      1520
TCTGCCTTCTGCTGATGCCCCATGTTCGTTATGGGTGTCAACAACAAGGAATACAACAAGACCGACATCAACGTCCCTC
SerAlaProSerAlaAspAlaProMetPheValMetGlyValAsnAsnLysGluTyrLysThrAspIleAsnValLeuSe

        1530      1540      1550      1560      1570      1580      1590      1600
TAACGCTTCTTGCACCAACTGCCTCCCCTTGCTAAGGTTATCAACGACAACTTCGGTCTCGTTGAGGGTCTCA
rAsnAlaSerCysThrThrAsnCysLeuAlaProLeuAlaLysValIleAsnAspAsnPheGlyLeuValGluGlyLeuM
```

To be continued to Fig. 2(c)

10

*Fig. 2(c)*

```
1610          1620          1630          1640          1650          1660          1670          1680
TGACCACTGTCCACTCCTACACTGCTACCCAGAAGACTGTCGATGCTCCCTCCGCCAAGGACTGGCGTGGGGACGTACC
etThrThrValHisSerTyrThrAlaThrGlnLysThrValAspAlaProSerAlaLysAspTrpArgGlyGlyArgThr

1690          1700          1710          1720          1730          1740          1750          1760
GCCGCTCAGAACATCATCCCCAGCTCCACTGGTGTGCTGCCAAGGGTCATTCCTTCTTCTTAACGGCAAGCT
AlaAlaGlnAsnIleIleProSerSerThrGlyAlaAlaAlaLysGlyIleProSerLeuAsnGlyLysLe

1770          1780          1790          1800          1810          1820          1830          1840
CACTGGTATGTCCATGCGGTGTGCCACCGCCAACGTCTCTGTTGTCGACCTCACCTGCCGTACCGAGAAGGCCGTCACCT
uThrGlyMetSerMetArgValProThrAlaAsnValSerValValAspLeuThrCysArgThrGluLysAlaValThrT

1850          1860          1870          1880          1890          1900          1910          1920
ACGAGGACATCAAGAAGACCATCAAGGCTGCTTCCGAGGAGGGCGGAGCTCAAGG GTAAGCACAAATACTGTCTTAGTTGCA
yrGluAspIleLysLysThrIleLysAlaAlaSerGluGluGlyGluGluLeuLysG

1930          1940          1950          1960          1970          1980          1990          2000
TGTATTTCGATATTTACTTAATTGGTTAG GCATTCTTGGATACACTGAGGACGACATTGTCTCCACTGACCTGATCGGA
                              IyIleLeuGlyTyrThrGluAspAspIleValSerThrAspLeuIleGly

2010          2020          2030          2040          2050          2060          2070          2080
GATGCCCACTCCTCCATCTTCGATGCCAAGGCCGGTATCGCCCTCAACGAGCACTTCATCAAGCTCGTCTCTTGGTACGA
AspAlaHisSerSerIlePheAspAlaLysAlaGlyIleAlaLeuAsnGluHisPheIleLysLeuValSerTrpTyrAs

2090          2100          2110          2120          2130          2140          2150          2160
CAACGAGTGGGGCTACTCCGCCGTGTTGTTGACCTCATTG GTAAGTCATTCCAGGCTTGAATCTGGGCTTTCCGGACCC
AsnGluTrpGlyTyrSerArgArgValValAspLeuIleA

2170          2180          2190          2200          2210          2220          2230          2240
TTACTAACGGTTCTCTTTTAG CCTACATCTCCAAGGTCGATGGCCAGTAGGAATCAGGACAGAGACCAGAAACTAGAAGT
                      laTyrIleSerLysValAspGlyGln###
```

To be continued to Fig. 2(d)

11

**Fig-2(f)**

GTTCTATAAACAAAACACTCTAGTCCCCTGACTGTGTCCCAAGTTCCCGGATTATCAGGTCACCACCCCTTGAAATTGAC

CTGTGGGATAACAAAGTGCCCTGTTGACCGGGGATCTACATATCGCATAAAAAGCATATGATAACGTACTTATTTAATGA

AATGATTTGACGTTTATACTGAGTAAAAGTCATACCTGACAAGTGCTTCCTTCCGCTAAATTTGAATACGTCCTAGCGTA

TTCATCGAGGAGTAGTCTCAATCTTAGGTGGTCTAGAATAGTTCCAGGATATCCATATACAGAGAGTTATACTGTCTTGG

CTAGCCTTGTAGGGTGATGGGTCTCGGCGAAGTACGGGAAATTCTCGGACGAGCGCAAATGATTTCAGGAAATAGTCATG

GCGGGAGATAGACAATTGAAAAACTTTTCAATCTTTATCCTAATTTCCAATTCAATCCGTCTTCGTTACTCATGTGAACC

AATATCAAGCCTAATGGTGGTTAGGTTTTCTGCCGTTTGCTGCAAGTGACCTGTTTGGTATCTATGACTAGCTGTAGTCA

CGTGCGGAANNTAAGTAAACCACGCTTTTTGGGGCTTCTCCAGGGAGCAGAGTACATAATCCAAGAACTCCTAAACGTGC

GCCAAAACACCTCTACTGGTGTGCACGTAGTAGATCAACCTTTATCTGTGGCTCTTCAGGGTTGGTGAGGATTCTATTTC

TTTCTCGAACATCTGTCTTGGATTGCCCTGGTCTGGTCTAGCACAAGCTGTCTTCGTCGTTGAGCTTCTAGGCTTTCGAA

CTACTAGTATTAAGTAGTTCGCTCCACAGTTGTGGTTTGATATCCCTCAACGCCAGCCGCCTTTATCGTTCTGCCTTATG

TCCTAGTACAGTTTGTGCCTTTTGTTTCAATCCTTCGTCACTTATTTGTTACTGTACTGTCCATTTTACTCTTTTTTATG

TCTTTTGCGCTGAATCCTTATTGGTCACTTGTCGTGTTGTTCTTTTATCGACGGGGTGTCTTGCCCAGGGACGCTCGGGG

TCTTGTGAGACTGACGTAATAGCCCAAGACGACAGGCTTGGCTATGGCATGC

Fig-3(a)

P    Hp          St St    Sc

ATG

pMT011

Sm

pUC118

HpaI/StuI
digestion

Hp          St

SmaI digestion

ligation

E        promoter              H

ATG

pGAP001

[Site-directed Mutagenesis]

MetAlaThrPro
wild type; ACACATCTAAACA ATGGCTACCCCC
              **    *
 mutant  ; ACGGATCCAAACA ATGGCTACCCCC
              Bam HI        MetAlaThrPro
                          *   **
primer; 5'-TAGCCATTGTTTGGATCCGTCTGTTGGTGT-3'

To be continued to Fig. 3(b)

EP 0 436 858 A1

# Fig-3 (b)

preparation of pGAP004
P;PstI, Hp;HpaI, St;StuI
Sc; SacI, Sm;SmaI, E;EcoRI
H;HindIII, Ba;BalI, Sp;SphI
Hc;HincII, B;BamHI

# Fig. 4 (a)

[Site-directed Mutagenesis]

```
                                                    MetLeuGlnSer
wild type; GAAAGGGGGATTTTATGCGTGAGA ATGTTACAGTCT
                           ** *
mutant; GAAAGGGGGATTGGATCCGTGAGA ATGTTACAGTCT
                       Bam HI          MetLeuGlnSer
                           ** *
primer; 5'-GAAAGGGGGATTGGATCCGTGAGAATGT-3'
```

To be continued to Fig. 4(b)

# Fig- 4 (b)

BamHI        B

**pBLE003**

E        B

promoter        terminator

**pGAP004**

BamHI
digestion        ligation        BamHI
digestion

B        B

promoter      BLE. REGIST.     terminator

**pGAP005**

preparation of pGAP005
( He; Hae III, B; BamHI, Hc; HincII )
( E; EcoRI )

16

European
Patent Office

EUROPEAN SEARCH
REPORT

Application Number

EP 90 12 3877

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | GENE, vol. 69, 1988, pages 49-57, Elsevier Science Publishers B.V. (Biomedical Division), Amsterdam, NL; P.J. PUNT et al.: "Isolation and characterization of the glyceraldehyde-3-phosphate dehydrogenase gene of Aspergillus nidulans" | | C 12 P 21/02 C 12 N 15/80 |
| A | GENE, vol. 48, nos. 2-3, 1986, pages 211-217, Elsevier Science Publishers B.V. (Biomedical Division), Amsterdam, NL; R.F.M. VAN GORCOM et al.: "A system for the analysis of expression signals in Aspergillus" | | |
| A | TRENDS IN BIOTECHNOLOGY, vol. 7, no. 10, October 1989, pages 283-287, Elsevier Science Publishers Ltd (UK), Cambridge, GB; G. SAUNDERS et al.: "Heterologous gene expression in filamentous fungi" | | |
| A | SCIENCE, vol. 244, 16th June 1989, pages 1313-1317, Washington, D.C., US; W.E. TIMBERLAKE et al.: "Genetic engineering of filamentous fungi" | | |
| A | WO-A-8 606 097 (ALLELIX INC.) | | |

TECHNICAL FIELDS
SEARCHED (Int. Cl.5)

C 12 P
C 12 N

The present search report has been drawn up for all claims

| Place of search | Date of completion of search | Examiner |
|---|---|---|
| The Hague | 04 April 91 | VAN PUTTEN A.J. |